# EUROPEAN PATENT APPLICATION

(11) **EP 3 824 898 A1**
(43) Date of publication of application: **26.05.2021**
(21) Application number: 20208448.9
(22) Date of filing: 10.03.2015
(51) Int. Cl.: A61K 36/752, A61K 36/28, A61K 31/375, A61P 3/06

(54) **DEHYDRATED EXTRACTS BASED PHYTO COMPLEX COMPRISING CITRUS BERGAMIA AND ITS PHARMACEUTICAL USE**

(30) Priority: 10.03.2014 IT RM20140111
(62) Divisional of application: 15715836.1
(71) Applicant: Esserre Pharma Società a Responsabilità Limitata, 00191 Rome (IT)
(72) Inventor: Riccioni, Costanza Valentina, 00191 Roma (IT); Squillace Greco, Amedeo, 00191 Roma (IT)
(74) Representative: Turri, Elisa

(57) **Abstract**

A phytocomplex based on dried extracts of Citrus Bergamia Risso et Poiteau, of Cynara scolymus L., L-ascorbic acid and phytosterols for medical use, as a supplement and in association with pharmacological therapies for reducing cholesterol and triglycerides.

## Description

### Field of the invention

The present invention refers to a particular dry extract of bergamot characterized by a high titration of flavonoids and other dry extracts of natural derivation. Said composition has shown hypocholesterolemizing and hypolipemizing properties of superior effectiveness compared with the components thereof considered individually. The use of bergamot extract, titrated in the flavonoids responsible for the hypocholesterolemizing action, and the presence of substances of proven effectiveness, such as phytosterols, artichoke and ascorbic acid, make this phytocomplex effective in the treatment of mild and moderate dyslipidaemia. The solution can be considered both from the perspective of traditional supplementation typical of the nutraceutical field and from the perspective of a possible support to pharmacological therapy.

### Prior art

To date, the interest in bergamot (Citrus Bergamia Risso et Poiteau) has regarded, in addition to the extraction of the essence from the epicarp, the activity of the polyphenols in the juice in maintaining physiological blood cholesterol, triglycerides and glucose levels. In Citrus Bergamia Risso et Poiteau juice, flavonoid compounds called brutieridin and melitidin, exclusive to this species of the Citrus genus, have been discovered (1).

These compounds are derived from the flavonoids hesperetin and naringenin (aglycones). In particular, they are derivatives of the glycosylated (neohesperidosides) forms of hesperetin and naringenin, which are called neohesperidin and naringin, respectively.

Brutieridin and melitidin possess a 3-hydroxy-3-methylglutaryl (HMG) portion bound to the glycosidic portion of the flavonoid.

Brutieridin is the name of the 3-hydroxy-3-methylglutaryl compound derived from neohesperidin; melitidin is the name of the 3-hydroxy-3-methylglutaryl compound derived from naringin.

The presence, in these flavonoids, of the 3-hydroxy-3-methylglutaryl portion is responsible for a statin-like hypocholesterolemizing action, through the inhibition of the enzyme hydroxymethylglutaryl coenzyme A reductase (HMG-CoA reductase) on the natural substrate (HMG CoA) (Leopoldini M et al., J, Agric. Food Chem. 2010, 58, 10768-10773).

This mechanism of action does not provoke competition at the active site between the polyphenols of bergamot and the drugs of choice for hypercholesterolemia (statins), enabling them to be used concomitantly. The association of bergamot juice and statin was demonstrated in a clinical study which highlighted the synergistic action thereof.(2)

International patent application WO2010/055490 refers to a process for producing a phytocomplex consisting of a dry extract of the bergamot fruit albedo and the use thereof in the pharmaceutical field as an antidyslipemic and/or antiatherogenic agent and as a dietary supplement. In particular, the phytocomplex obtained with the disclosed process is characterized by the following percentages of bioflavonoids:
Neoeriocitrine 29.6 % +/- 6.0,
Naringine 32.4 % +/- 4.0,
Neoesperidine 38.0 % +/- 6.0
and has a minimum content of the listed flavonoids not lower than 25%.

Miceli N., Journal of Agricultural and Food Chemistry, 2007, 55, 10671-10677 discloses that the administration of C. Bergamia juice to hyperlipidemic mice provides a reduction of cholesterol, triglycerides and LDL, together with an increase in HDL in serum.

A further study (5) describes the administration of C. Bergamia juice in both animal models and in humans. The study on humans was conducted on 237 patients, distinguished into groups of patients with isolated hypercholesterolaemia, mixed hyperlipidaemia (high levels of cholesterol and triglycerides) and metabolic syndrome (patients with high levels of cholesterol, triglycerides associated with hyperglycaemia) who were treated daily for 30 days with bergamot juice or with a placebo. Treatment with the polyphenolic fraction derived from C. Bergamia juice led to a reduction in total cholesterol and LDL, triglycerides and glycaemia (depending on group) and to an increase in HDL. The groups treated with the placebo did not obtain appreciable results. The polyphenolic fraction of bergamot used contained 26-28% of the 5 main flavonoids. The content of the 5 main flavonoids was:
neoeriocitrin (7.7%±0.4%), naringin (6.3%±0.33%), neohesperidin (7.2%±0.35%), melitidin (1.56%±0.11%) and brutieridin (3.32%±0.17%).

International patent application WO01/24805 refers to a composition comprising at least one component of vegetal origin, e.g. Citrus limonum Risso, Cynara Scolymus L. and a compound selected from vitamin C, vitamin E and beta-carotene, capable of lowering hyperlipidaemia. Citrus limonum Risso does not comprise the flavonoids melitidin and brutieridin, which are exclusive to bergamot and characterize the hypocholesterolemizing and hypolipemizing activities thereof.

Phytosterols (in common language the term indicates plant sterols and stanols) are lipophilic compounds of the triterpene family, bioactive components of plant cell membranes. They are characterized by a chemical structure similar to that of cholesterol, made up of a tetracyclic ring and a long flexible side chain at the carbon atom C-17, but differ from the latter in the identity of the lateral chain at C-17.

Plant stanols are characterized by the absence of the double bond Δ-5 on the sterolic ring.

Plant sterols and stanols are not endogenously synthesized in humans, but are taken in through the diet.

Natural sources of phytosterols are, for example, vegetable oils, cereals, nuts, vegetables and fruit.

Different mechanisms have been identified which, by acting on different phases, can explain the hypocholesterolemizing effect of phytosterols.

Firstly, by virtue of their greater lipophilism, phytosterols have a greater affinity than cholesterol for incorporation into mixed micelles.

The reduction in the concentration of micellar cholesterol has the consequence of a lower uptake of cholesterol by enterocytes.

Secondly, phytosterols interfere with the normal mechanisms responsible for maintaining the intracellular homeostasis of cholesterol, which depends on various factors, including the activity of ABC transporters.

Recent studies have shown, in fact, that phytosterols are capable of activating Liver X nuclear receptors in enterocytes, thus increasing the cholesterol efflux mediated by ABC transporters in the intestinal lumen. Since the two transporters ABCG5/ABCG8 are present in the liver as well, it may be hypothesized that the beneficial effect of phytosterols on cholesterol is also due to an increase in its excretion in bile.

Numerous clinical studies have shown a decrease in LDL cholesterol levels of around 8-9% correlated to the intake of products (functional foods and food supplements) with added phytosterols (2g/day).(3) However, there have been conflicting studies, according to which, in healthy subjects, a low or high intake of plant sterols had no influence on plasma concentrations of LDL cholesterol despite the modulation of the metabolism of cholesterol (Lin X, et al., Eur J Clin Nutr 2010; 64; 1481-7). Other studies suggest that triglyceride levels are reduced by 6-4% with the intake of 1.6-2.5 g/day of plant stanols (Demonty I., et al., Eur Jç Nutr 2013; 52; 153-60; Naumann E. et al., J Am Coll Nutr 2008, 27: 117-26).

Relevant data have been obtained from clinical studies which evaluated the association of products supplemented with phytosterols to statin-based pharmacological therapy.

The results show, with the association of phytosterols and statins, a further 10-15% decrease in LDL cholesterol levels, greater than the one obtained by doubling the dose of statin (6%).(3) This evidence suggests an effect of phytosterols that is added to the one exerted by statins, thanks to the difference in the mechanisms of action with which these molecules act, the former (phytosterols) inhibiting intestinal absorption and the latter (statins) intervening in cholesterol biosynthesis.

The artichoke (Cynara scolymus L.) belongs to the family Asteraceae (Compositae) and grows in the Mediterranean basin and in countries with a temperate climate.

The dry extract of the leaves is characterized by the presence of organic acids, phenolic acids, including chlorogenic acid (5-caffeoylquinic acid) and flavonoids: one in particular, luteolin, to which the hypocholesterolemizing activities of the drug are attributed. This seems to have an indirect mechanism of inhibition on HMGCoA-reductase, as it inhibits the processes activating the enzymatic system; its inhibitory effectiveness on the HMG-CoA-reductase enzymatic system activated after insulin-dependent stimulation was also demonstrated. For this reason, it is recommended to take the phytocomplex containing artichoke, among other substances, during or after the main meals, when the secretion of insulin and thus the activation of HMG-CoA reductase are at a maximum.

The hypocholesterolemizing effect is also due to another characteristic compound of the artichoke: chlorogenic acid. The structure-activity relationship has been amply demonstrated in various clinical studies which have served to confirm the antioxidant activity on LDL, till to a reduction in LDL cholesterol levels of 23% in few weeks.(4)

A further study describes a 4.2% reduction in total cholesterol obtained after 12 weeks with a daily dosage of 1280 mg of a standardized broad-spectrum aqueous extract of artichoke leaves (4-6:1) in healthy adults with mild to moderate hypercholesterolemia. No significant differences were observed in LDL cholesterol levels, HDL or triglycerides (Bundy R et al., Phytomedicine, 2008, 15 (9): 668-75). In addition to the antioxidant and cholesterol biosynthesis-inhibiting properties, there are choleretic and colagogic ones, whereby intrahepatic cholesterol is eliminated thanks to its excretion through bile acids.

Such effects have proven to be interesting, as they contribute to preventing the formation of atherosclerotic plaque and the events associated with it.

Vitamin C (L-ascorbic acid or ascorbic acid) is a strong reducing agent which, by virtue of its antioxidant properties, is involved in preventing the damaging effects of free radicals.

The role of vitamin C in the synthesis of collagen, neurotransmitters and carnitine is well known.

Ascorbic acid is an enzymatic cofactor and increases gastrointestinal absorption of non-heme iron.

Experimental studies have highlighted the inhibitory activity of vitamin C on lipid peroxidation, which is important in the prevention of atherosclerosis.

### Detailed description of the invention

A phytocomplex has now been found which comprises a Citrus Bergamia Risso et Poiteau extract having hypocholesterolemizing and hypolipemizing properties of superior efficacy compared with the components thereof considered individually.

The object of the present invention is thus a phytocomplex characterized in that it comprises a Citrus Bergamia Risso et Poiteau extract and at least one substance selected from the group consisting of: Cynara scolymus L. extract, L-ascorbic acid and phytosterols.

Said phytocomplex is preferably characterized in that it comprises Citrus Bergamia Risso et Poiteau extract, Cynara scolymus L. extract, L-ascorbic acid and phytosterols.

Said phytocomplex is preferably characterized in that said extracts are dehydrated.

Even more preferably, said phytocomplex is characterized in that said extracts are dehydrated in the form of micronized, lyophilized or granulated particles.

In a preferred embodiment of the invention, the Citrus Bergamia Risso et Poiteau extract contains a titration in total flavonoids not lower than 60% w/w relative to the total of dried extract of Citrus Bergamia Risso et Poiteau juice. Preferably, the Citrus Bergamia Risso et Poiteau extract comprises at least 7% w/w of brutieridin and melitidin relative to the total fraction of flavonoids thereof. Even more preferably, in the phytocomplex according to the invention, the Citrus Bergamia Risso et Poiteau extract comprises about 30% w/w of brutieridin and melitidin relative to the total fraction of flavonoids thereof.

More preferably, the Citrus Bergamia Risso et Poiteau extract comprises the following percentages w/w of flavonoids:
- brutieridin 17.5%,
- melitidin 12.7%,
- naringin 30.6%,
- neohesperidin 27.2%,
- eriocitrin 12%,
total 100%

The phytocomplex according to the invention is preferably in tablet, capsule, granulate, liquid or semiliquid beverage form. The capsules are preferably made of vegetable gelatine.

Preferably, the phytocomplex as defined above comprises an amount of Citrus Bergamia Risso et Poiteau extract of at least 200 mg and/or an amount of phytosterols of at least 120 mg and/or an amount of Cynara scolymus L. extract of at least 80 mg and/or an amount of L-ascorbic acid of at least 20 mg.

More preferably, the phytocomplex as defined above comprises an amount of Citrus Bergamia Risso et Poiteau extract comprised between 200 mg and 400 mg and/or an amount of phytosterols comprised between 120 and 240 mg and/or an amount of Cynara scolymus L. extract comprised between 80 and 160 mg and/or an amount of L-ascorbic acid of between 20 and 40 mg.

Even more preferably, the phytocomplex as defined above comprises an amount of Citrus Bergamia Risso et Poiteau extract of 200 mg and an amount of phytosterols of 120 mg and an amount of Cynara scolymus L. extract of 80 mg and an amount of L-ascorbic acid of 20 mg.

Preferably, the phytocomplex as described above consists of an amount of Citrus Bergamia Risso et Poiteau extract of 200 mg and an amount of phytosterols of 120 mg and an amount of Cynara scolymus L. extract of 80 mg and an amount of L-ascorbic acid of 20 mg.

In an alternative embodiment, the phytocomplex as defined above comprises an amount of Citrus Bergamia Risso et Poiteau extract of 400 mg and an amount of phytosterols of 240 mg and an amount of Cynara scolymus L. extract of 160 mg and an amount of L-ascorbic acid of 40 mg.

The phytocomplex according to the invention is preferably in association with antidyslipidaemic or hypolipidemizing pharmaceutical compounds, more preferably in association with at least one statin and/or ezetimibe.

Another object of the invention is a pharmaceutical composition comprising the phytocomplex as described above and at least one pharmaceutically acceptable excipient.

A further object of the invention is a dietary supplement comprising the phytocomplex as described above.

Further objects of the invention are the phytocomplex as defined above or the pharmaceutical composition as defined above, for medical use, preferably in the treatment and/or prevention of dyslipidaemia, preferably mixed dyslipidaemia (hypercholesterolemia and hypertriglyceridemia), hypercholesterolemia, preferably familial or polygenic, hypertriglyceridemia, diabetes associated with dyslipidaemia, metabolic syndrome, statin-induced myalgia or myopathy, intolerance to hypolipidemizing drugs, clinical conditions characterized by low HDL cholesterol levels or for the prevention of the atherosclerosis.

Another object of the invention is the use of the phytocomplex as described above or of the supplement as described above in the nutraceutical field.

Another object of the invention is a method for the treatment and/or prevention of dyslipidaemia, preferably mixed dyslipidaemia (hypercholesterolemia and hypertriglyceridemia), hypercholesterolemia, preferably familial or polygenic, hypertriglyceridemia, diabetes associated with dyslipidaemia, metabolic syndrome, statin-induced myalgia or myopathy, intolerance to hypolipidemizing drugs or clinical conditions characterized by low HDL cholesterol levels or in the prevention of atherosclerosis, comprising the administration, in a subject in need therefor, of an effective amount of the phytocomplex as defined above or of the pharmaceutical composition as defined above.

The Citrus Bergamia Risso et Poiteau extract according to the invention is a dry or dried extract of Citrus Bergamia Risso et Poiteau juice.

In the Citrus Bergamia Risso et Poiteau extract, the flavonoids brutieridin and melitidin preferably constitute about 30% w/w of the total flavonoid fraction of the extract.

The process of obtaining the dry or dried extract of Citrus Bergamia Risso et Poiteau juice must be such that said extract has the above-described characteristics.

The Cynara scolymus L. extract is preferably in the form of a dry extract from artichoke leaves, more preferably titrated at 2.5% w/w in chlorogenic acid on the total of the extract itself.

The phytosterols in the phytocomplex are preferably derived from soy (Glycine max. L.). They are preferably in powder form, even more preferably in 95% pure powder form. Preferably, the plant sterols present and the percentage amount thereof in the phytosterol powder are: beta-sitosterol (>40%), campesterol (>20%), stigmasterol (>15%), brassicasterol (20%).

Preferably, in the phytocomplex as described above, the phytosterols are titrated at least at 95% w/w relative to the total of the soy-derived phytosterol powder.

The phytocomplex as described above can further comprise at least one substance selected from the group consisting of: omega 3, vitamin D, calcium, folic acid, iron, astaxasthin, berberine, coenzyme Q and magnesium.

The phytocomplex according to the invention can comprise two or more of the above-described components and all the possible combinations thereof.

In the phytocomplex as described above, the components thereof are preferably dehydrated or dry or dried or in powder form.

Another object of the invention is a process for obtaining the phytocomplex as described above, comprising the addition to the Citrus Bergamia Risso et Poiteau extract, of at least one substance selected from the group consisting of: Cynara scolymus L. extract, L-ascorbic acid, phytosterols.

Preferably, the administration of the phytocomplex can take place, for example, in the form of tablets with the addition of excipients, vegetable gelatine capsules with the addition of excipients, of granular sachets or of liquid or semiliquid beverage.

Preferably, the administration of the phytocomplex or of the pharmaceutical composition according to the invention takes place in doses of bergamot extract comprised between 200 and 800 mg per day, of phytosterols between 120 and 480 mg per day, of artichoke extract between 80 and 320 mg and of vitamin C between 20 and 80 mg.

More preferably, the administration takes place in doses of 400 mg per day of bergamot extract, 240 mg per day of phytosterols, 160 mg of artichoke extract and 40 mg of vitamin C.

The dosage units of the phytocomplex or pharmaceutical compositions according to the invention can contain the above-described amounts or submultiples thereof in order to reach the daily dose. The choice of a dosage for a particular patient is well known to the person skilled in the art. The compositions are normally accompanied by instructions for use in the treatment in question.

In the context of the present invention, the definition of "phytocomplex" also comprises, but is not limited to, terms such as composition, mixture, etc.

In the context of the present invention, the Citrus Bergamia Risso et Poiteau extract can be indicated as bergamot extract.

In the context of the present invention, the Cynara scolymus L. extract can be indicated as artichoke extract.

The phytocomplex object of the present invention allows to obtain a reduction in total cholesterol, in LDL cholesterol and in triglycerides and an increase in HDL cholesterol, with greater values than values obtained using the components thereof individually.

The pharmaceutical composition can be chosen on the basis of the treatment requirements. Such pharmaceutical compositions or the phytocomplex according to the invention can be administered in the form of tablets, capsules, oral preparations, powders, granules, pills, injectable, or infusible liquid solutions, suspensions, suppositories, preparation for inhalation.

Tablets and capsules for oral administration are normally presented in unit dose form and contain conventional excipients such as binders, fillers (including cellulose, mannitol, lactose), diluents, tableting agents, lubricants (including magnesium stearate), detergents, disintegrants (e.g. polyvinylpyrrolidone and starch derivatives such as sodium glycolate starch), coloring agents, flavoring agents, and wetting agents (for example sodium lauryl sulfate).

Oral liquid preparations can be in, for example, aqueous form in oily suspensions, solutions, emulsions, syrups, or can be presented as a dry product for reconstitution with water. The liquid preparations can contain conventional additives such as suspending agents, emulsifying agents, non-aqueous vehicles

A reference for the formulations is the book by Remington ("Remington: The Science and Practice of Pharmacy", Lippincott Williams & Wilkins, 2000).

The phytocomplex or the pharmaceutical compositions as above defined may be administered in a single dosage containing all the components of the phytocomplex or as separate compositions (simultaneous or sequential) of the individual components. The pyhtocomplex may be administered in combination with other active ingredients which may be separately formulated into single-ingredient preparations of one of the above-described forms and then administered as combined preparations, which are given at the same time or different times, or may be formulated together into the same preparation.

The expert in the art will select the form of administration and effective dosages by selecting suitable diluents, adjuvants and / or excipients.

Some examples of pharmaceutical forms or pharmaceutical compositions comprising the phytocomplex object of the invention are shown below.

Example 1: A phytocomplex preparation in the form of swallowable tablets was prepared, containing:
- bergamot (Citrus Bergamia Risso et Poit., dry extract of juice) 200 mg
- soy phytosterols (Glycine max. L., 95% powder) 120 mg
- artichoke (Cinara Scolymus L., dry extract of leaves) 80 mg
- vitamin C (L-ascorbic acid) 20 mg

### Excipients:

- sorbitol
- maize maltodextrin
- microcrystalline cellulose
- magnesium stearate
- silicon dioxide
q.s. to a total of: 700 mg

### Example 2:

A phytocomplex preparation in the form of swallowable type 0 capsules was prepared, containing:
- bergamot (Citrus Bergamia Risso et Poit., dry extract of juice) 300 mg
- soy phytosterols (Glycine max. L., 95% powder) 200 mg
- artichoke (Cinara Scolymus L., dry extract of leaves) 90 mg

### Excipients:

- gelatine
- glycerine
- titanium dioxide
- microcrystalline cellulose
- colloidal silica
q.s. to a total of: 1000 mg

### Example 3:

A phytocomplex preparation in the form of swallowable type 0 capsules was made containing:
- bergamot (Citrus Bergamia Risso et Poit., dry extract of juice) 400 mg
- soy phytosterols (Glycine max. L., 95% powder) 240 mg
- artichoke (Cinara Scolymus L., dry extract of leaves) 160 mg
- vitamin C (L-ascorbic acid) 40 mg

### Excipients:

- gelatine
- glycerine
- titanium dioxide
- microcrystalline cellulose
- colloidal silica
q.s. to a total of: 1000 mg

### Example 4:

A phytocomplex preparation in the form of granulate contained in sachets and to be dissolved in water was made, containing:
- bergamot (Citrus Bergamia Risso et Poit., dry extract of juice) 500 mg
- soy phytosterols (Glycine max. L., 95% pure powder) 600 mg
- artichoke (Cinara Scolymus L., dry extract of leaves) 100 mg
- vitamin C (L-ascorbic acid) 60 mg

### Excipients:

- fructose
- sodium citrate tribasic dihydrate
- saccharine
- water-soluble beta-carotene 10%
- calcium carbonate
- calcium phosphate tribasic
- citric acid monohydrate
- polysorbate 80
- maltol
- sweet orange essence
- orange essence
- sucralose
q.s. to a total of: 5000 mg

The association with pharmaceutical compounds, in particular with statins, is especially recommended in long-term therapies.

The composition object of the present invention, in the form of dry extracts, can be used in the nutraceutical field, for example in tablet form with the addition of at least one excipient, or it can be encapsulated in capsules of vegetable gelatine with the addition of at least one pharmaceutically acceptable excipient.

The particle size of each ingredient enables the pharmacokinetic and pharmacodynamic characteristics to be improved, increasing their bioavailability, by micronizing the extracts, for example. The production of lyophilized or granulates enables the administration of the phytocomplex in sachets for oral use. These can also be used as semi-finished preparations for the production of capsules, tablets or coated granules.

The presence of a water-soluble portion enables it to be used, for example, in liquid and semiliquid forms.

Considering the high titration in active substances and their antioxidant and anti-inflammatory properties, the phytocomplex as described above finds application in the nutraceutical field, for example as an anti-aging agent, for the prevention of the tumours.

Added to the by now well-documented synergistic action of the phytocomplex in question there is a synergistic action deriving from the association between the phytocomplex and the pharmacological therapy, in particular with statins, the drugs of choice for treating dyslipidaemia.

At the site of action level, this synergism is explained by the different mechanisms of inhibiting cholesterol biosynthesis on the enzymatic system of HMG-CoA reductase; these do not create competition at the active site, either between the bergamot and the drug, or between the phytosterols and the bergamot. This synergism is further confirmed by the synergistic effects between the phytosterols present in the phytocomplex and the drug.

The present invention will now be described in non-limiting examples thereof.

### Examples

### Materials and Methods

### Preparation of the phytocomplex

The botanical name of the bergamot extract used is Citrus Bergamia Risso et Poiteau. This extract is derived from the juice of the fruit. Citrus Bergamia Risso et Poit. juice is obtained by mechanical squeezing of fresh, very ripe fruit devoid of rot and visible mould.

The raw materials used neither contain nor derive from genetically modified organisms.

In a subsequent step the juice is depulped, pasteurized and collected in aseptic receptacles. The obtained juice then undergoes a process of filtration, extraction and concentration.

In particular, the juice is passed through specific membranes and subjected to successive steps of microfiltration and nanofiltration, which enable the passage of water, ions and water-soluble compounds (such as flavonoids) while excluding the passage of microorganisms such as viruses and bacteria.

The next process consists in passing the filtered juice through specific non-polymeric adsorbent resins (to avoid the release of low-molecular-weight polymers, potentially harmful to health) which make it possible to obtain an extract of Citrus Bergamia Risso et Poit. juice that is very rich in flavonoids.

Finally, the juice obtained is subjected to a spray-drying process which allows to eliminate water and to obtain a dry extract. The extract titration is then determined by HPLC (high performance liquid chromatography). The flavonoid titration is described in detail below, with regard in particular to two flavonoids (exclusive to Citrus Bergamia Risso et Poiteau), which are responsible for the hypolipemizing activity (brutieridin and melitidin) and which constitute about 30% of the flavonoid fraction of the extract:
- brutieridin 17.5%,
- melitidin 12.7%,
- naringin 30.6%,
- neohesperidin 27.2%,
- eriocitrin 12%,
total 100%

The glycosylated portion enables the active ingredients to pass easily through the cells; the covalent bond between a glycoside and the phenolic portion increases the water solubility and bioavailability and protects against degradation.

The phytosterols used in the phytocomplex are derived from soy (Glycine max. L.) and are in the form of a 95% pure powder. In particular, the type of plant sterols present and the percentage amount thereof in the phytosterol powder are as follows: beta-sitosterol (>40%), campesterol (>20%), stigmasterolo (>15%) and brassicasterol (20%).

To the dose of Citrus Bergamia Risso et Poit. (200 mg) it is added the dose of phytosterols (120 mg) titrated of at least 95% w/w relative to the total of the powder, the artichoke (80 mg) (dry extract titrated at 2.5% w/w in chlorogenic acid relative to the total of the extract itself), which has known cholagogic and choleretic properties and ascorbic acid (20 mg) for its well-known antioxidant properties. The same indicated dosages can be duplicated for each component.

### Clinical study n. 1

The synergistic association was tested on a group of 200 patients in a randomized, double-blind, placebo-controlled clinical study. The study was approved by the hospital ethics committee. The patients included in the study were of both sexes (104 males and 96 females), aged between 35 and 75 years, who were affected by familial hypercholesterolemia (i.e. due to genetic factors) or polygenic hypercholesterolemia (i.e. due to the interaction among a number of factors such as diet, lifestyle and environment, together with genetic factors) and hypertriglyceridemia, with total cholesterol values comprised between 220 and 300 mg/dl.

The duration of the study was three months.

Before and after the study, the patients underwent a clinical evaluation and blood chemistry tests (total cholesterol, HDL, LDL, triglyceride, creatinine and glycaemia, GOT, GPT, CPK). The patients were randomized and divided into two groups:
- group A (105 patients) was treated with one capsule per day of the phytocomplex containing 400 mg of dry extract of Citrus Bergamia Risso et Poit. juice, 240 mg of soy phytosterols, 160 mg of artichoke leaf extract and 40 mg of vitamin C, plus excipients (coating: gelatine and glycerine; diluents and glidants: maltodextrin, titanium dioxide, colloidal silica)
- group B (95 patients) was treated with one capsule per day of placebo, containing exclusively excipients (coating: gelatine and glycerine; diluents and glidants: maltodextrin, titanium dioxide, colloidal silica).

After 30 days of treatment, the patients in group A showed an evident reduction in the levels of total cholesterol, LDL and triglycerides and a significant increase in the HDL cholesterol levels.
- in all patients in group A, a reduction comprised between 34 and 42% in total cholesterol, a reduction comprised between 35 and 43% in LDL cholesterol and a reduction of between 38 and the 48% in triglyceride were obtained, whilst an increase comprised between 32 and the 40% was obtained in HDL cholesterol.

After 90 days of treatment the above-described results were confirmed.

These data can be thus exemplified: a male patient who prior to the treatment had values of total cholesterol of 280 mg/dl, LDL cholesterol of 160 mg/dl, HDL of 35 mg/dl and triglycerides of 240 mg/dl reached, after the treatment, total cholesterol values that can be comprised, according to the percentage of reduction, between 184 and 162 mg/dl, LDL cholesterol between 104 and 91 mg/dl, triglycerides between 148 and 124 mg/dl and HDL cholesterol between 46 and 49 mg/dl.

This means that the patient reached physiological values across the entire lipid profile.

In contrast, the patients of group B showed no appreciable variations in their lipid profile.

No patient reported any side effects as a result of the treatment either with the phytocomplex or the placebo.

### Results

In light of the different mechanisms action which characterize the ingredients contained in the phytocomplex, very interesting results (in vivo) on the total cholesterol, LDL, HDL and triglyceride levels were, obtained as they were superior to the already known hypolipemizing actions of the components considered individually.

In the study conducted with the phytocomplex object of the present invention, the results obtained in the 105 patients affected with mixed dyslipidaemia (familial or polygenic hypercholesterolemia and hypertriglyceridemia) treated with the phytocomplex were superior to the ones known for the individual substances.

In particular, the following results were obtained:
- a reduction in total cholesterol comprised between 34 and 42%
- a reduction in LDL cholesterol comprised between 35 and 43%
- a reduction in triglycerides comprised between 38 and 48%
- an increase in HDL cholesterol comprised between 32 and 40%

These data confirm the synergistic action of the substances within the phytocomplex.

The obtained results were in fact compared with the data in the literature regarding the hypocholesterolemizing effects of phytosterols and artichoke (3, 4, Bundy et al., 2008) as well as with those of a clinical study which involved an exclusively treatment with a dry extract titrated in polyphenols of bergamot.(5, WO2010/055490) The composition of the extract of Citrus Bergamia Risso et Poit., phytosterols, artichokes and vitamin C showed superior results compared both to phytosterols and artichokes taken individually and compared to the treatment with bergamot extract alone.

### Clinical study n. 2

A second clinical study was conducted on 60 female patients aged between 40 and 75 years, 30 of whom were affected by anti-aromatase arthralgia (group A) and 30 affected by statin-induced myopathy (group B).

The patients in both groups had a lipid profile characterized by high levels of total cholesterol and LDL cholesterol and low HDL cholesterol levels.

In particular:
- The patients in group A had total cholesterol values comprised between 210 and 260 mg/dl (with an average of 240 mg/dl), values of LDL cholesterol between 110 and 150 mg/dl (with an average of 120 mg/dl), whereas they had values of HDL cholesterol comprised between 37 and 49 mg/dl (with an average of 45 mg/dl) and triglycerides between 150 and 200 mg/dl (with an average of 170 mg/dl).
- The patients in group B had total cholesterol values comprised between 250 and 310 mg/dl (with an average of 280 mg/dl), values of LDL cholesterol between 110 and 170 mg/dl (with an average of 150 mg/dl), values of HDL cholesterol between 36 and 44 mg/dl (with an average of 40 mg/dl) and triglycerides between 150 and 220 mg/dl (with an average of 190 mg/dl).

Before starting the therapy with the phytocomplex, the patients underwent a washout of the hypolipemizing drugs previously taken and during the treatment with the phytocomplex they took no other drugs or substances with hypolipemizing activity.

All patients were administered the phytocomplex in the form of swallowable tablets, each of which containing 200 mg of dry extract of Citrus Bergamia Risso et Poit. juice, 120 mg of soy phytosterols, 80 mg of artichoke leaf extract and 20 mg of vitamin C.

After six months of treatment with two tablets per day of the phytocomplex, in both groups excellent results were obtained in reducing the levels of total cholesterol, LDL and triglycerides.

No patient reported or showed side effects as a result of treatment with the phytocomplex.
- The patients in group A showed on average a 27% reduction in the levels of total cholesterol, a 29% reduction in LDL cholesterol and a 35% reduction in triglycerides.

This means the attainment of physiological values of total cholesterol (from a average value of 240 mg/dl to an average value of 175 mg/dl) and of LDL (from an average value of 120 mg/dl to an average value of 85 mg/dl) and of triglycerides (from an average value of 170 mg/dl to an average value of 110 mg/dl)
- The patients in group B showed on average a 37% reduction in the levels of total cholesterol, a 36% reduction in LDL cholesterol and a 40% reduction in triglycerides.

This means the attainment of physiological values of total cholesterol (from an average value of 280 mg/dl to an average value of 176 mg/dl) and of LDL (from an average value of 150 mg/dl to an average value of 96 mg/dl) and triglycerides (from an average value of 190 mg/dl to an average value of 114 mg/dl).

In particular, the results on the increase in HDL in the treated patients proved to be very interesting.

The lipid profile of the female population is closely related to the endogenous production of estrogens and for this reason women in post-menopause often have an altered lipid profile, with high LDL cholesterol levels and low HDL cholesterol levels.

Treatment with aromatase inhibitors, antitumour drugs which inhibit the biosynthesis of estrogens, has been correlated with a decrease in HDL cholesterol levels.

HDL cholesterol is an important parameter for assessing cardiovascular risk in the male and above all female population, where low HDL cholesterol levels are to a larger degree an indicator of an increased cardiovascular risk than high LDL cholesterol levels.

In particular, in women an HDL cholesterol level greater than 50 mg/dl is recommended.

After six months of treatment with two tablets per day of the phytocomplex, the patients in group A obtained on average a 33% increase in HDL cholesterol (thus the levels increased on average from a value of 45 mg/dl to a value of 60 mg/dl), whilst the patients in group B obtained on average a 38% increase in that parameter (thus the levels increased on average from a value of 40 mg/dl to a value of 55 mg/dl).

This result appears to be of great interest if compared with data related to the increase in HDL cholesterol in patients undergoing treatment with statins, the hypolipemizing drugs that are most prescribed in absolute terms.

In studies conducted on patients with high LDL cholesterol levels and HDL cholesterol levels that were normal for the sex (40-50 mg/dl for men; 50-60 mg/dl for women), a 5 to 10% increase was observed in HDL cholesterol levels, irrespective of the dosage or the statin administered.

However, in patients with low HDL cholesterol levels (<35 mg/dl), statins can differ in their effect on HDL cholesterol levels.

Simvastatin, at a maximum dose of 80 mg, is capable of increasing HDL cholesterol levels to a larger extent than a comparable dose of atorvastatin. In preliminary studies conducted on hypertriglyceridaemic patients with low HDL-C, rosuvastatin seems to increase HDL cholesterol by as much as 15-20% (Goodman and Gilman's The Pharmacological Basis of Therapeutics. 12th Ed.(2011)).

In light of these data, it may be inferred that the action of the phytocomplex in increasing HDL cholesterol levels by 33-38% is important for the purposes of treating patients with an altered lipid profile, in particular in menopausal women and women with particular clinical conditions.

### Clinical study n. 3

In order to evaluate the effectiveness of the composition of the present invention and the synergistic action thereof in association with a pharmacological therapy, a clinical study was conducted on 33 patients affected by familial or polygenic hypercholesterolaemia.

A group of 16 patients was treated with an association of the phytocomplex (two tablets per day) and 10mg of a statin (rosuvastatin). A group of 17 patients instead took 20 mg of rosuvastatin. After a month of treatment, a 39% (tChol), 53% (cLDL) and 46% (TG) decrease and a 38% increase (cHDL) were recorded. These results were the same as those obtained after a month of treatment with a double dose of the statin in question (20 mg of rosuvastatin) without the association with the phytocomplex. These data demonstrate the synergistic action of the phytocomplex plus statins, and represent a valid alternative to long-term treatment with the statin alone in subjects with intolerance toward the drug.

### References

1. Di Donna L, De Luca G., Mazzotti F, Napoli A, Salerno R, Taverna D, and Sindona G., (2009). "Statin-like Principles of Bergamot Fruit (Citrus bergamia): Isolation of 3-Hydroxymethylglutaryl Flavonoid Glycosides". J. Nat. Prod. 72, 1352-1354.
2. Micaela Gliozzi, Ross Walker, Saverio Muscoli, Cristiana Vitale, Santo Gratteri, Cristina Carresi, Vincenzo Musolino, Vanessa Russo, Elzbieta Janda, Salvatore Ragusa, Antonio Aloe, Ernesto Palma, Carolina Muscoli, Franco Romeo, Vincenzo Mollace, (2013). "Bergamot polyphenolic fraction enhances rosuvastatin-induced effect on LDL-cholesterol, LOX-1 expression and protein kinase B phosphorylation in patients with hyperlipidemia".International Journal of Cardiology 170, Issue 2, 140- 145.
3. Helena Gylling, Jogchum Plat, Stephen Turley, Henry N. Ginsberg, Lars Ellegård, Wendy Jessup, Peter J. Jones, Dieter Lütjohann, Winfried Maerz, Luis Masana, Giinther Silbernagel, Bart Staels, Jan Boren, Alberico L. Catapano, Guy De Backer, John Deanfield, Olivier S. Descamps, Petri T. Kovanen, Gabriele Riccardi, Lale Tokgozoglu, M. John Chapman, (2014)."Plant sterols and plant stanols in the management of dyslipidaemia and prevention of cardiovascular disease". The European Atherosclerosis Society Consensus Panel on Phytosterols. Atherosclerosis 232, 346-360.
4. Englisch W, Beckers C, Unkauf M, Ruepp M, Zinserling V. Arzneim.-Forsch, (2000). Efficacy of artichoke dry extract in patients with hyperlipoproteinemia. Drug Res.;50:260-265.
5. Mollace V, Sacco I, Janda E, Malara C, Ventrice D, Colica C, Visalli V, Muscoli S, Ragusa S, Muscoli C, Rotiroti D, Romeo F. (2011). "Hypolipemic and hypoglycaemic activity of bergamot polyphenols: From animal models to human studies". Fitoterapia; 82:309-16.

Embodiments of the present invention are as follows:
1. A phytocomplex characterized in that it comprises a Citrus Bergamia Risso et Poiteau extract and at least one substance selected from the group consisting of: Cynara scolymus L. extract, L-ascorbic acid, phytosterols.
2. The phytocomplex according to embodiment 1, characterized in that it comprises Citrus Bergamia Risso et Poiteau extract, Cynara scolymus L. extract, L-ascorbic acid and phytosterols.
3. The phytocomplex according to embodiment 1 or 2, characterized in that said extracts are dehydrated in the form of micronized, lyophilized or granulated particles.
4. The phytocomplex according to any one of the preceding embodiments, characterized in that the Citrus Bergamia Risso et Poiteau extract contains a titration in total flavonoids not lower than 60% w/w relative to the total of dried extract of Citrus Bergamia Risso et Poiteau juice.
5. The phytocomplex according to any one of the preceding embodiments, characterized in that the Citrus Bergamia Risso et Poiteau extract comprises at least 7% w/w of brutieridin and melitidin relative to the total fraction of flavonoids thereof.
6. The phytocomplex according to embodiment 5, wherein the Citrus Bergamia Risso et Poiteau extract comprises about 30% w/w of brutieridin and melitidin relative to total fraction of flavonoids thereof.
7. The phytocomplex according to embodiment 5 or 6, wherein the Citrus Bergamia Risso et Poiteau extract comprises the following percentages w/w of flavonoids:
   - brutieridin 17.5%,
   - melitidin 12.7%,
   - naringin 30.6%,
   - neohesperidin 27.2%,
   - eriocitrin 12%,
   total 100%
8. The phytocomplex according to any one of the preceding embodiments, in tablet, capsule, granulate, liquid or semiliquid beverage form.
9. The phytocomplex according to embodiment 8, comprising an amount of Citrus Bergamia Risso et Poiteau extract of at least 200 mg and/or an amount of phytosterols of at least 120 mg and/or an amount of Cynara scolymus L. extract of at least 80 mg and/or an amount of L-ascorbic acid of at least 20 mg.
10. The phytocomplex according to embodiment 9 comprising an amount of Citrus Bergamia Risso et Poiteau extract comprised between 200 mg and 400 mg and/or an amount of phytosterols comprised between 120 and 240 mg and/or an amount of Cynara scolymus L. extract comprised between 80 and 160 mg and/or an amount of L-ascorbic acid of between 20 and 40 mg.
11. The phytocomplex according to embodiment 9 or 10, comprising an amount of Citrus Bergamia Risso et Poiteau extract of 200 mg and an amount of phytosterols of 120 mg and an amount of Cynara scolymus L. extract of 80 mg and an amount of L-ascorbic acid of 20 mg.
12. The phytocomplex according to any one of the preceding embodiments, in association with antidyslipidaemic or hypolipidemizing pharmaceutical compounds, preferably in association with at least one statin and/or ezetimibe.
13. A pharmaceutical composition comprising the phytocomplex according to any one of embodiments 1-12 and at least one pharmaceutically acceptable excipient.
14. A dietary supplement comprising the phytocomplex according to any one of embodiments 1-11.
15. The phytocomplex as defined in any one of embodiments 1-12 or a pharmaceutical composition as defined in embodiment 13, for medical use, preferably in the treatment and/or prevention of dyslipidaemia, preferably mixed dyslipidaemia (hypercholesterolemia and hypertriglyceridemia), hypercholesterolemia, preferably familial or polygenic, hypertriglyceridemia, diabetes associated with dyslipidaemia, metabolic syndrome, statin-induced myalgia or myopathy, intolerance to hypolipidemizing drugs, clinical conditions characterized by low HDL cholesterol levels or for the prevention of the atherosclerosis.
16. Use of the phytocomplex as disclosed in any one of embodiments 1-12 or of the supplement described in embodiment 14 in the nutraceutical field.
17. A method for the treatment and/or prevention of dyslipidaemia, preferably mixed dyslipidaemia (hypercholesterolemia and hypertriglyceridemia), hypercholesterolemia, preferably familial or polygenic, hypertriglyceridemia, diabetes associated with dyslipidaemia, metabolic syndrome, statin-induced myalgia or myopathy, intolerance to hypolipidemizing drugs or clinical conditions characterized by low HDL cholesterol levels or in the prevention of atherosclerosis, comprising the administration, in a subject in need therefor, of an effective amount of the phytocomplex as defined in any one of embodiments 1-12 or of the pharmaceutical composition as defined in embodiment 13.

## Claims

1. A phytocomplex **characterized in that** it comprises a Citrus Bergamia Risso et Poiteau extract and at least one substance selected from the group consisting of: Cynara scolymus L. extract, L-ascorbic acid, phytosterols.

2. The phytocomplex according to claim 1, wherein the at least one substance is Cynara scolymus L. extract.

3. The phytocomplex according to claim 1, wherein the at least one substance is phytosterols.

4. The phytocomplex according to claim 1, **characterized in that** it comprises Citrus Bergamia Risso et Poiteau extract, Cynara scolymus L. extract, L-ascorbic acid and phytosterols.

5. The phytocomplex according to any one of previous claims, **characterized in that** said extracts are dehydrated in the form of micronized, lyophilized or granulated particles.

6. The phytocomplex according to any one of the preceding claims, **characterized in that** the Citrus Bergamia Risso et Poiteau extract contains a titration in total flavonoids not lower than 60% w/w relative to the total of dried extract of Citrus Bergamia Risso et Poiteau juice.

7. The phytocomplex according to any one of the preceding claims, **characterized in that** the Citrus Bergamia Risso et Poiteau extract comprises at least 7% w/w of brutieridin and melitidin relative to the total fraction of flavonoids thereof,
preferably wherein the Citrus Bergamia Risso et Poiteau extract comprises about 30% w/w of brutieridin and melitidin relative to total fraction of flavonoids thereof.

8. The phytocomplex according to claim 7, wherein the Citrus Bergamia Risso et Poiteau extract comprises the following percentages w/w of flavonoids:
- brutieridin 17.5%,
- melitidin 12.7%,
- naringin 30.6%,
- neohesperidin 27.2%,
- eriocitrin 12%,
total 100%.

9. The phytocomplex according to any one of the preceding claims, in tablet, capsule, granulate, liquid or semiliquid beverage form.

10. The phytocomplex according to claim 9, comprising an amount of Citrus Bergamia Risso et Poiteau extract of at least 200 mg and/or an amount of phytosterols of at least 120 mg and/or an amount of Cynara scolymus L. extract of at least 80 mg and/or an amount of L-ascorbic acid of at least 20 mg,
preferably comprising an amount of Citrus Bergamia Risso et Poiteau extract comprised between 200 mg and 400 mg and/or an amount of phytosterols comprised between 120 and 240 mg and/or an amount of Cynara scolymus L. extract comprised between 80 and 160 mg and/or an amount of L-ascorbic acid of between 20 and 40 mg,
preferably comprising an amount of Citrus Bergamia Risso et Poiteau extract of 200 mg and an amount of phytosterols of 120 mg and an amount of Cynara scolymus L. extract of 80 mg and an amount of L-ascorbic acid of 20 mg.

11. The phytocomplex according to any one of the preceding claims, in association with antidyslipidaemic or hypolipidemizing pharmaceutical compounds, preferably in association with at least one statin and/or ezetimibe.

12. A pharmaceutical composition comprising the phytocomplex according to any one of claims 1-11 and at least one pharmaceutically acceptable excipient.

13. A dietary supplement comprising the phytocomplex according to any one of claims 1-10.

14. The phytocomplex as defined in any one of claims 1-11 or a pharmaceutical composition as defined in claim 12, for medical use, preferably in the treatment and/or prevention of dyslipidaemia, preferably mixed dyslipidaemia (hypercholesterolemia and hypertriglyceridemia), hypercholesterolemia, preferably familial or polygenic, hypertriglyceridemia, diabetes associated with dyslipidaemia, metabolic syndrome, statin-induced myalgia or myopathy, intolerance to hypolipidemizing drugs, clinical conditions **characterized by** low HDL cholesterol levels or for the prevention of the atherosclerosis.

15. Use of the phytocomplex as disclosed in any one of claims 1-11 or of the supplement described in claim 13 in the nutraceutical field.
